# EUROPÄISCHE PATENTSCHRIFT

(11) **EP 0 920 871 B1**
(45) Veröffentlichungstag und Bekanntmachung des Hinweises auf die Patenterteilung: **01.12.1999**
(21) Anmeldenummer: 97114376.3
(22) Anmeldetag: 20.08.1997
(51) Int. Cl.: A61K 35/78

(54) **Verfahren zur Verhinderung von Ausfällungen in frischen Presssäften von Echinacea**
Process for inhibiting precipitation in fresh press juice from echinacea
Procédé pour empêcher les précipitations dans les jus pressés d'echinacea

(43) Veröffentlichungstag der Anmeldung: 09.06.1999
(73) Patentinhaber: Plantamed Arzneimittel GmbH, 92318 Neumarkt (DE)
(72) Erfinder: Joseph, Heinz Walter, 71522 Backnang (DE)
(74) Vertreter: Sandmair, Kurt, Dr. Dr.

(56) Entgegenhaltungen:
- DE-A- 2 721 014
- DE-A- 4 438 589

## Beschreibung

Die vorliegende Anmeldung betrifft ein Verfahren zur Verhinderung von Ausfällungen in frischen Preßsäften von Echinacea. Weiterhin betrifft die Anmeldung stabilisierte Echinacea-Preßsäfte sowie diese enthaltende Arzneiformen.

Echinacea-Präparate werden innerlich als Urologika, Grippemittel, Antiphlogistika und Umstimmungsmittel verwendet. Äußerlich werden sie zur Wundbehandlung angewandt.

Echinacea-Zubereitungen, insbesondere in Form von Tinkturen und Preßsäften sind schon lange in Gebrauch und stellen ein wohlbekanntes Naturheilmittel dar. Ebensolange wie ihr Gebrauch ist die Neigung der Pflanzenpreßsäfte zur Bildung von Ausfallungen bekannt. Um dieses Problem in den Griff zu bekommen, läßt man den Preßsaft üblicherweise über einen sehr langen Zeitraum, meist ca. zwei Jahre, stehen, und filtriert den währenddessen gebildeten Niederschlag anschließend zur Gewinnung eines klaren Pflanzenpreßsaftes ab.

Dennoch neigen auch diese klaren Filtrate trotz der ersten Ausfällung während der zweijährigen Lagerzeit nach Abfüllung in die verkaufsfertigen Flaschen weiterhin zur Bildung von Ausfällungen und Ausflockungen, was zu einer negativen Compliance der Patienten führt. Außerdem besteht die Möglichkeit, daß durch die gebildeten Ausfällungen die Tropfer der Flaschen verstopfen oder verengt werden und somit eine exakte Dosierung nicht mehr möglich ist. Beide Erscheinungen führen häufig zur Rücksendung des Präparats an den Hersteller als verdorben oder nicht mehr brauchbar.

Aus der DE-A-27 21 014 sind beispielsweise Arzneimittel bekannt, die rechtsdrehende Milchsäure (Salze) sowie ein Pulver von Echinacea purpurrea oder Echinacea angustipholia enthalten. Das Arzneimittel liegt als Pulver vor und kann in Form von Tabletten, Lutschbonbons oder Kapseln verabreicht werden.

Weiterhin ist aus der DE-A-44 38 589 ein Arzneimittel, speziell ein Nasentherapeutikum gegen Virusinfektionen und allergische Krankheiten bekannt, das als Wirkstoffe Preßsäfte, Extrakte oder eine Urtinktur aus dem Kraut oder den Wurzeln von Echinaceapflanzen enthält. Zur Stabilisierung werden dem Preßsaft 22 Vol.-% Alkohol zugesetzt. Das Problem von Ausflockungen wird nicht beschrieben.

Darüber hinaus ergeben sich aufgrund der langen Lagerzeit der frischen Preßsäfte vor der Abfüllung in die verkaufsfertigen Dosiseinheiten Kapazitäts- und Kostenprobleme für den Hersteller.

Es ist nun Aufgabe der vorliegenden Erfindung ein Verfahren bereitzustellen, das die Ausfällungen verhindert bzw. eine frühere Abfüllung des Preßsaftes in verkaufsfertige Dosiseinheiten ermöglicht und somit das Problem sowohl der schlechten Patienten-Compliance als auch das Problem der langen Lagerzeiten löst.

Überraschenderweise wurde nun gefunden, daß sich die extrem lange Lagerzeit von zwei Jahren vermeiden läßt, indem man den frischen Pflanzenpreßsaft kurzzeitig auf Temperaturen unter 0 °C kühlt, die Lösung filtriert und durch Säurezusatz stabilisiert. Durch dieses Vorgehen läßt sich der gewonnene, frische Pflanzenpreßsaft deutlich stabilisieren, so daß auch nach mehreren Monaten Lagerzeit keine Trübung oder Ausfällung auftritt.

Weiterhin überwindet das erfindungsgemäße Verfahren die Notwendigkeit, den Pflanzenpreßsaft über lange Zeit zu lagern und so die erste Ausfällung praktisch abzuwarten. Demgegenüber sind die Preßsäfte sofort zu verarbeiten, erfordern keinen Lagerraum mehr, und sind wesentlich stabiler als dies bei den herkömmlichen Präparaten der Fall ist.

Erfindungsgemäß wird der frische Preßsaft über einen kürzeren Zeitraum von 14 Tagen oder weniger, vorzugsweise 1 bis 5 Tagen und noch stärker bevorzugt 2 bis 3 Tagen kühl gelagert. Die Lagerungstemperatur liegt erfindungsgemäß zwischen 0 und - 15 °C, vorzugsweise -5 bis - 10 °C und am meisten bevorzugt bei -8 °C.

Nach der kühlen Lagerung wird der Pflanzenpreßsaft klar filtriert. Die Filtration kann mittels herkömmlicher, üblicherweise bei Pflanzenpreßsäften angewandter Verfahren z. B. mittels Sieb- oder Papierfiltern, Cellulosefiltern, Membranen wie auch über Zentrifugation und anderer zur Abtrennung von Feststoffen aus Lösungen geeigneter Prozesse erfolgen.

Nach Filtration des zuvor gekühlten Pflanzenpreßsafts wird dieser durch Säurezusatz auf pH-Werte von <pH 6,0 eingestellt. Vorzugsweise liegt der eingestellte pH-Wert im Bereich von 4,5 bis 5,3 und noch stärker bevorzugt im Bereich von 4,9 bis 5,0.

Die zur Einstellung des pH-Werts verwendete Säure ist eine natürlich vorkommende Carbonsäure, vorzugsweise eine polyvalente Carbonsäure, die zudem physiologisch verträglich sein muß. Derartige Säuren umfassen, sind jedoch nicht beschränkt auf, Weinsäure, die natürlich vorkommenden Aminosäuren und die Säuren des Citrat-Zyklus, insbesondere Citronensäure, Äpfelsäure, Fumarsäure, Itaconsäure, Aconitsäure, Bernsteinsäure, Milchsäure, Glutaminsäure, Asparaginsäure, Oxalsäure und Ascorbinsäure. Besonders bevorzugt sind Äpfelsäure, Fumarsäure und Citronensäure, wobei letztere am meisten bevorzugt ist.

Der Zusatz der Säure erfolgt in der zur Einstellung des pH-Werts erforderlichen Menge, was von Säurestärke sowie dem Ausgangs-pH-Wert des einzustellenden Präparats abhängig ist. Üblicherweise werden 0,01 bis 5 % Säure, vorzugsweise 0,05 bis 1 % Säure und am meisten bevorzugt 0,1 % (w/v) Säure zugesetzt.

Bei dem genannten Pflanzenpreßsaft kann es sich um Tinkturen, Fluidextrakte und Pflanzenpreßsäfte sowie diese enthaltende Zubereitungen handeln. Besonders bevorzugt handelt es sich um eine ethanolische Preßsaftzubereitung, enthaltend 20 bis 60 % Echinacea-Preßsaft, noch stärker bevorzugt eine 80 % Echinacea-Preßsaft enthaltende ethanolische Zubereitung.

### Beispiel:

Zur Stabilisierung wurde ein Echinacea-Pflanzenpreßsaft, der auf einen Ethanolendgehalt von 20 % stabilisiert wurde, drei Tage auf -8 °C gekühlt, und anschließend über einen Cellulosefilter zur Klärung abfiltriert. Die erhaltene klare Lösung wurde durch Zusatz von 0,1 Citronensäure auf einen pH-Wert von 4,9 eingestellt.

Die fertige Lösung wurde in Flaschen abgefüllt und diese zur Beobachtung bei Raumtemperatur gelagert. Das erhaltene Produkt ist über lange Zeit (mehr als 24 Monate) stabil, es bilden sich keine Ausfällungen.

Im Vergleich hierzu wurde ein gemäß dem herkömmlichen Verfahren über zwei Jahre gelagerter Pflanzenpreßsaft klar filtriert und in Flaschen abgefüllt. Dieses Präparat zeigte bereits nach zwei Monaten eine deutliche Trübung und ein dementsprechend für den Patienten weniger ansprechendes Äußeres.

## Patentansprüche

1. Verfahren zur Verhinderung von Ausfällungen in Echinacea-Pflanzenpreßsäften bei dem man den frisch gewonnenen Pflanzenpreßsaft für 14 Tage oder weniger auf Temperaturen zwischen 0 und -15 °C kühlt, die gekühlte Lösung klar filtriert und mit natürlich vorkommender Carbonsäure auf einen pH-Wert unter 6 einstellt.

2. Verfahren gemäß Anspruch 1, bei dem die Säure ausgewählt ist unter Citronensäure, Weinsäure, Äpfelsäure, Fumarsäure, Itaconsäure, Aconitsäure, Bernsteinsäure, Milchsäure, Glutaminsäure, Asparaginsäure, Oxalsäure und Ascorbinsäure.

3. Verfahren nach Anspruch 3, bei dem die Säure Citronensäure ist.

4. Verfahren gemäß Anspruch 1, bei dem die Säure in einer Menge von 0,01 bis 5 % (w/v) zugegeben wird.

5. Verfahren gemäß einem der vorstehenden Ansprüche, bei dem der Echinacea-Pflanzenpreßsaft für zwei bis drei Tage auf -8°C gekühlt, anschließend filtriert und durch Zusatz von 0,1 % Citronensäure auf einen pH-Wert von 4,9 eingestellt wird.

6. Stabilisierter Pflanzenpreßsaft aus Echinacea, herstellbar nach ein der Verfähren gemäß Anspruch 1 bis 5.

7. Stabilisierter Pflanzenpreßsaft gemäß Anspruch 6, enthaltend 0,1 % Citronensäure.

8. Arzneimittelzubereitung enthaltend einen Pflanzenpreßsaft gemäß einem der Ansprüche 6 oder 7.

## Claims

1. A method of preventing precipitation in pressed echinacea plant juice, wherein the freshly prepared pressed plant juice is cooled for 14 days or less to temperatures of between 0 and -15°C, the cooled solution is filtered clear and is adjusted to a pH value of below 6 with naturally occurring carboxylic acid.

2. A method according to Claim 1, wherein the acid is chosen from citric acid, tartaric acid, malic acid, fumaric acid, itaconic acid, aconitic acid, succinic acid, lactic acid, glutamic acid, aspartic acid, oxalic acid and ascorbic acid.

3. A method according to Claim 3 [sic], wherein the acid is citric acid.

4. A method according to Claim 1, wherein the acid is added in a quantity of 0.01 to 5 % (w/v).

5. A method according to any one of the preceding Claims, wherein the pressed echinacea plant juice is cooled for two to three days to -8°C, is subsequently filtered and is adjusted to a pH value of 4.9 by the addition of 0.1 % citric acid.

6. A stabilised pressed plant juice of echinacea which can be prepared in accordance with one of the methods according to Claims 1 to 5.

7. A stabilised pressed plant juice according to Claim 6, containing 0.1 % citric acid.

8. A pharmaceutical preparation containing a pressed plant juice according to either Claim 6 or Claim 7.

## Revendications

1. Procédé destiné à empêcher les précipitations dans les jus pressés d'echinacea, dans lequel on refroidit le jus pressé fraîchement obtenu pendant 14 jours ou moins, à une température comprise entre 0 et -15°C, on filtre le jus refroidi pour le clarifier et on ajuste son pH à une valeur inférieure à 6, à l'aide d'un acide carboxylique naturel.

2. Procédé selon la revendication 1, dans lequel l'acide est choisi parmi l'acide citrique, l'acide tartrique, l'acide malique, l'acide fumarique, l'acide itaconique, l'acide aconitique, l'acide succinique, l'acide lactique, l'acide glutamique, l'acide aspartique, l'acide oxalique et l'acide ascorbique.

3. Procédé selon la revendication 3, dans lequel l'acide est l'acide citrique.

4. Procédé selon la revendication 1, dans lequel l'acide est ajouté en une proportion de 0,01 à 5 % (p/v).

5. Procédé selon l'une quelconque des revendications précédentes, dans lequel on refroidit le jus pressé d'echinacea pendant deux à trois jours à -8°C, on le filtre ensuite et on ajuste son pH à 4,9 par addition de 0,1 % d'acide citrique.

6. Jus pressé d'echinacea stabilisé, pouvant être obtenu par le procédé selon l'une quelconque des revendications 1 à 5.

7. Jus pressé stabilisé selon la revendication 6, contenant 0,1 % d'acide citrique.

8. Préparation pharmaceutique contenant un jus pressé selon la revendication 6 ou 7.
